# EUROPEAN PATENT APPLICATION

(11) **EP 2 944 277 A1**
(43) Date of publication of application: **18.11.2015**
(21) Application number: 13871287.2
(22) Date of filing: 10.01.2013
(51) Int. Cl.: A61B 17/11, A61B 17/00

(54) **APPLICATOR**

(71) Applicant: Terumo Kabushiki Kaisha, Tokyo 151-0072 (JP)
(72) Inventor: FUKUI, Kunio, Ashigarakami-gun Kanagawa 259-0151 (JP); YOKOYAMA, Kenji, Fujinomiya-shi Shizuoka 418-0015 (JP)
(74) Representative: Casalonga
(86) International application number: PCT/JP2013/050298
(87) International publication number: WO 2014/109031

(57) **Abstract**

An applicator is provided with a nozzle which includes a nozzle head located at the tip thereof and an inner part to which gas is fed, at least two inner tubes which are inserted into the nozzle and used for feeding at least two kinds of liquids that gel when mixed together to the nozzle head, and a liquid feed unit which feeds each of the liquids to each of the inner tubes. The nozzle head includes a mixing unit body to which the inner tubes are connected and inside which the liquids are allowed to join and mixed together and an opening which is formed on the tip side of the mixing unit body and used for jetting a liquid mixture of the liquids together with the gas. The gas is fed from at least the rear end side with respect to a confluent section in which the liquids join together in the mixing unit body.

## Description

### Technical Field

The present invention relates to an applicator which is preferably used in, for example, laparoscopic surgery and applies, for example, an antiadhesive material and a biological tissue adhesive to a target region such as the affected part, and particularly, to an applicator which does not cause clogging even when an antiadhesive material and a biological tissue adhesive are intermittently applied to the affected part.

### Background Art

There is currently proposed an applicator which uses two or more kinds of liquids which gel when mixed together to form, for example, an antiadhesive material and a biological tissue adhesive for spraying the antiadhesive material and the biological tissue adhesive in a gel state to a target region such as the affected part (e.g., refer to Patent Literature 1).

In the applicator of Patent Literature 1, a combination of a first liquid and a second liquid which gel when mixed together is used. For example, the gelling of a liquid mixture obtained by mixing the first liquid and the second liquid together enables the liquid mixture to reliably remain in the target region, for example, the biological tissue or the affected part to which the liquid mixture is applied. Since the liquid mixture reliably remains in the target region, the liquid mixture reliably exhibits a function as a biological tissue adhesive and an antiadhesive material.

In the applicator of Patent Literature 1, a first inner tube and a second inner tube join together at the tip parts thereof to form a confluent section in which inner spaces of the inner tubes join together (refer to Fig. 23 of Patent Literature 1). The confluent section has a small diameter part which is located on the tip side and has a small inner diameter and a large diameter part which is located on the base end side and has a large inner diameter. Most part of the large diameter part of the confluent section includes a gas permeable film. The gel liquid mixture is jetted through a tip opening of the small diameter part together with gas. The gas is continuously fed.

In the applicator of Patent Literature 1, while the liquid mixture is jetted, gas that has passed through the gas permeable film is turned into micro bubbles in the liquid mixture passing through the confluent section. The liquid mixture is stirred by the micro bubbles in the course of passing through the confluent section. Accordingly, the first liquid and the second liquid are uniformly and reliably mixed together and sprayed as a liquid mixture. In particular, when the viscosities of the liquids are different from each other, a uniform liquid mixture is hardly obtained merely by allowing the liquids to join together. However, since the micro bubbles exhibit a stirring action of stirring the first liquid and the second liquid to accelerate the mixing of the first liquid and the second liquid, a uniform liquid mixture can be obtained.

### Citation List

### Patent Literature

Patent Literature 1: JP 2009-131590 A

### Summary of Invention

### Technical Problem

When the applicator of Patent Literature 1 is continuously used, the liquid mixture is discharged to the outside by the pressure of feeding the first liquid and the second liquid and gas. Thus, clogging does not occur.

However, when the applicator is intermittently used in such a manner that the liquid mixture is applied to a target region once and again applied to the target region after a certain time, the liquid mixture may remain and gel in the confluent section, which may cause clogging in the confluent section which includes a gas permeable film. Such clogging reduces the gas flow rate and increases force for pushing out the liquid mixture from a liquid feed unit. This disadvantageously makes it difficult to intermittently and repeatedly apply the liquid mixture to the target region.

An object of the present invention is to solve the problems based on the conventional techniques and provide an applicator capable of preventing clogging not only in continuous use, but also in intermittent use.

### Solution to Problem

In order to achieve the above object, the present invention provides an applicator which is provided with a nozzle including a nozzle head located at a tip thereof and an inner part to which gas is fed, at least two inner tubes inserted into the nozzle and used for feeding at least two kinds of liquids that gel when mixed together to the nozzle head, and a liquid feed unit feeding each of the liquids to each of the inner tubes, wherein the nozzle head includes a mixing unit body to which the inner tubes are connected and inside which the liquids are allowed to join and mixed together and an opening formed on a tip side of the mixing unit body and used for jetting a liquid mixture of the liquids together with the gas, and the gas is fed from at least a rear end side with respect to a confluent section in which the liquids join together in the mixing unit body.

Preferably, the mixing unit body is provided with a gas introduction section which allows the gas to pass therethrough and feeds the gas to the confluent section from at least the rear end side, the gas introduction section being located on the rear end side of the confluent section.

Preferably, the mixing unit body is further provided with a stirring section which allows the gas to pass therethrough and at least stirs the liquid mixture, the stirring section being located between the confluent section and the opening.

For example, the gas introduction section includes a tubular gas permeable member and an end on the rear end side of the gas introduction section is sealed. Further, the gas introduction section may include a member having at least one hole formed for allowing the gas to pass therethrough.

Preferably, the confluent section includes a tubular member, and the inner tubes are connected to a peripheral face of the confluent section at positions symmetric with respect to an axis in a longitudinal direction.

The confluent section may be formed of a material having a lower gas permeability than the gas permeable member.

### Advantageous Effects of Invention

The present invention makes it possible to prevent clogging not only in continuous use, but also in intermittent use.

### Brief Description of Drawings

Fig. 1 is a schematic view illustrating an applicator of an embodiment of the present invention.
Fig. 2(a) is a schematic view illustrating a mixing unit body of a nozzle head of the applicator of the embodiment of the present invention, Fig. 2(b) is a schematic view illustrating another example of the mixing unit body of the nozzle head of the applicator, and Fig. 2(c) is a schematic view illustrating still another example of the mixing unit body of the nozzle head of the applicator.
Fig. 3 is a schematic sectional view illustrating the configuration of a mixing unit body of a nozzle head of an applicator of Comparative Example 1.
Fig. 4 is a schematic view illustrating a measurement device used in a method of measuring the gas flow rate and the resistance in the applicator.

### Description of Embodiment

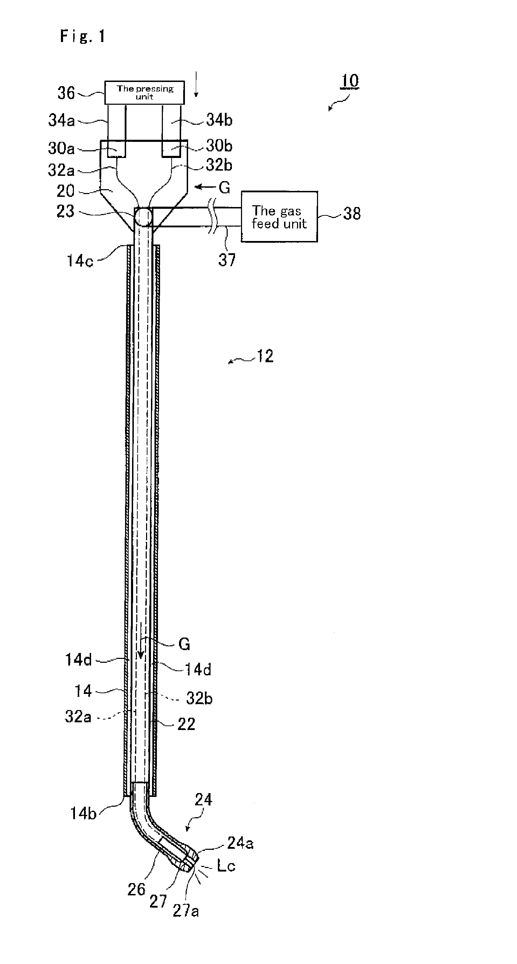

Hereinbelow, an applicator of the present invention will be described in detail on the basis of a preferred embodiment illustrated in the accompanying drawings. Fig. 1 is a schematic view illustrating the applicator of the embodiment of the present invention.

The applicator 10 includes a nozzle 12 and a sheath 14 into which the nozzle 12 is inserted. The nozzle 12 includes a spray head 20, a tubular nozzle body 22 having a base end to which the spray head 20 is connected, and a nozzle head 24 which is connected to the tip of the nozzle body 22.

The spray head 20 is provided with a first connection section 30a which is connected to a first syringe 34a and a second connection section 30b which is connected to a second syringe 34b.

An inner tube 32a for feeding a first liquid fed from the first syringe 34a to the nozzle head 24 is connected to the first connection section 30a. An inner tube 32b for feeding a second liquid fed from the second syringe 34b to the nozzle head 24 is connected to the second connection section 30b.

The first inner tube 32a and the second inner tube 32b are inserted into the nozzle body 22 and connected to a mixing unit body 26 of the nozzle head 24.

The inner diameter of the first inner tube 32a and the inner diameter of the second inner tube 32b may be equal to each other or different from each other, and may be appropriately determined depending on the kind and use of liquid to be used. The connection configuration of the first inner tube 32a and the second inner tube 32b to the mixing unit body 26 will be described below in detail.

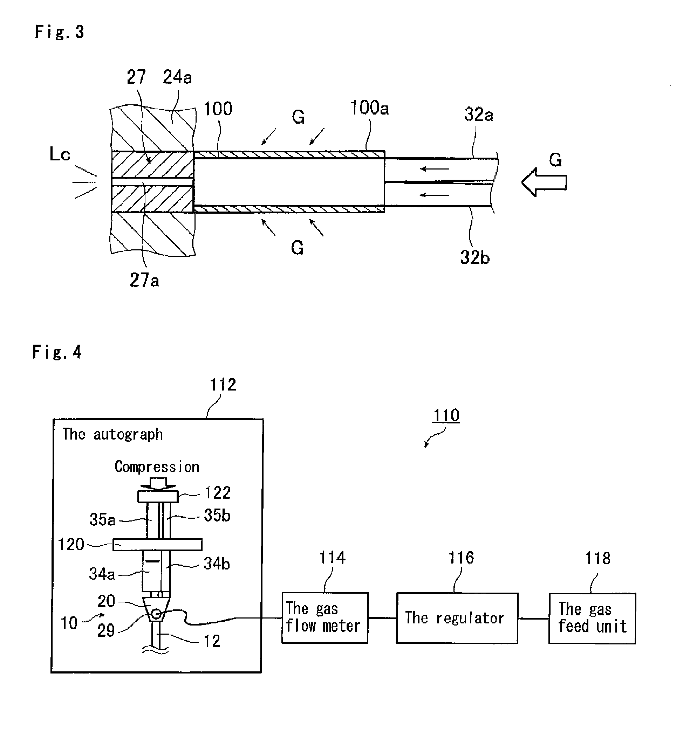

The first syringe 34a and the second syringe 34b respectively have a pusher 35a and a pusher 35b (not illustrated in Fig. 1, refer to Fig. 4). The pushers 35a and 35b are connected to a pressing unit 36. The first liquid and the second liquid are fed to the nozzle head 24 by pressing the pushers 35a and 35b by the pressing unit 36.

A syringe having a common configuration can be used as the first syringe 34a and the second syringe 34b. For example, a syringe described in JP 2009-131590 A or a syringe described in JP 2009-226189 A can be used. The configuration of the pressing unit 36 is not particularly limited to any configuration, and may be automatic or manual as long as it is capable of pressing the pushers 35a and 35b.

The first syringe 34a, the second syringe 34b, and the pressing unit 36 constitute a liquid feed unit.

As the first liquid filled in the first syringe 34a and the second liquid filled in the second syringe 34b, a combination of liquids which gel when mixed together is used. For example, a combination of an antiadhesive material and a biological tissue adhesive is used. The combination of the first liquid and the second liquid is appropriately selected depending on, for example, the use and intended purpose of the applicator 10, and case. The gelling of the mixture of the first liquid and the second liquid (hereinbelow, referred to as "liquid mixture") enables the liquid mixture to reliably remain in a target region to which the liquid mixture is applied.

The spray head 20 is provided with a port 23 which communicates with the nozzle body 22. A gas feed unit 38 is connected to the port 23 through a tube 37.

The gas feed unit 38 is used for feeding gas G in an aseptic condition to the nozzle head 24 through the nozzle body 22 serving as a gas flow passage at a predetermined gas flow rate to jet a liquid mixture Lc. The configuration of the gas feed unit 38 is not particularly limited to any configuration, and one used in a conventional applicator may be appropriately used. For example, the gas feed unit 38 is provided with a gas cylinder which is filled with the gas G, a valve which controls the feed/stoppage of the gas G, a flow meter which measures the flow rate of the gas G, and a regulator which regulates the feed pressure of the gas G. The gas flow rate of the gas G is, for example, 1 to 2 L/min. Such a flow rate enables the liquid mixture Lc to be jetted in a mist form. The gas G is desirably air or nitrogen.

The nozzle body 22 and the nozzle head 24 are relatively movably inserted into the sheath 14 along the longitudinal direction thereof. The nozzle body 22 includes, for example, a hollow stainless shaft. The sheath 14 includes, for example, a long tubular body made of polyethylene whose opposite ends are open. A clearance 14d between the sheath 14 and the nozzle body 22 functions as an exhaust passage. Gas from a tip 14b of the sheath 14 passes through the clearance 14d and is discharged to the outside through a rear end 14c of the sheath 14.

The nozzle head 24 is used for jetting the liquid mixture Lc to the outside from a tip 24a together with the gas G. The mixing unit body 26 and a tip member 27 which has an opening 27a are disposed inside the nozzle head 24.

In the following description, in the mixing unit body 26, a side facing the tip 24a of the nozzle head 24 is referred to as a tip side, and a side opposite to the tip 24a of the nozzle head 24 is referred to as a rear end.

The tip member 27 is disposed on the tip side of the mixing unit body 26. The tip member 27 is attached to the tip 24a of the nozzle head 24. The opening 27a of the tip member 27 corresponds to an opening of the nozzle head 24. The tip member 27 includes, for example, stainless steel. The tip member 27 may be integrated with the nozzle head 24.

In the mixing unit body 26, for example, the first liquid and the second liquid fed thereto are mixed together and stirred for gelling.

The mixing unit body 26 includes, for example, a tubular gas permeable member, that is, a gas permeable tube. The gas permeable tube is permeable to gas, but impermeable to liquid. The gas permeable tube is formed of, for example, a hydrophobic material. Specifically, a porous tube made of polytetrafluoroethylene (PTFE) is used.

The mixing unit body 26 has a confluent section 40, a stirring section 42, and a gas introduction section 44 (hereinbelow, also merely referred to as the introduction section 44).

The confluent section 40 is a region in which the first liquid fed through the first inner tube 32a and the second liquid fed through the second inner tube 32b join together. The first inner tube 32a and the second inner tube 32b are connected to a peripheral face 29 of the confluent section 40 at positions that are symmetric with respect to an axis C of the mixing unit body 26 in the longitudinal direction, that is, in an axial direction D.

The first inner tube 32a and the second inner tube 32b are preferably connected to the peripheral face such that respective ends thereof are flush with the inner face of the mixing unit body 26. This prevents or reduces convection of the first liquid and the second liquid in the confluent section 40 and prevents or reduces stagnation of the first liquid and the second liquid in the confluent section 40.

The stirring section 42 is a region in which the first liquid and the second liquid are stirred for gelling using the gas G at least from either the periphery or the rear end side. The stirring section 42 is located on the tip side of the confluent section 40 and provided between the confluent section 40 and the tip member 27.

The introduction section 44 is a region for allowing the gas G passing through the nozzle body 22 to permeate into the mixing unit body 26, and feeding the gas G from the rear end side of the confluent section 40 of the liquids and allowing the gas G to flow in the axial direction D with respect to the confluent section 40 to move the liquid mixture Lc and the first liquid and the second liquid in an unmixed state inside the mixing unit body 26 to the tip side so as to be discharged through the opening 27a. The introduction section 44 is located on the rear end side of the confluent section 40.

An end on the rear end side of the introduction section 44 is sealed by a sealing section 46. The gas G cannot pass through the sealing section 46. The configuration of the sealing section 46 is not particularly limited to any configuration.

The gas G that has passed through the mixing unit body 26 is turned into micro bubbles in the liquid mixture Lc inside the mixing unit body 26, so that a uniform liquid mixture Lc can be obtained.

In the applicator 10, the gas G flows into the confluent section 40 and the stirring section 42 in the above manner to mix the first liquid and the second liquid together and stir the first liquid and the second liquid for gelling during the jetting of the liquid mixture Lc. Further, the liquid mixture Lc is jetted through the opening 27a of the tip member 27 together with the gas G.

During the jetting, the gas G is continuously fed at a predetermined gas flow rate, and flow in the axial direction D is generated in the confluent section 40 by the gas G flowing into the confluent section 40 from the rear end side thereof. The flow in the axial direction D pushes out the entire flow passage in a direction perpendicular to the axial direction D of the mixing unit body 26. The first liquid and the second liquid are moved toward the tip side and discharged to the outside through the opening 27a by the flow in the axial direction D.

When the feed of the first liquid and the second liquid is stopped, the first liquid and the second liquid in an unmixed state and the liquid mixture Lc remaining inside the mixing unit body 26 are moved toward the tip side and discharged to the outside through the opening 27a by the flow of the gas G in the axial direction D from the rear end side. This prevents the remaining of the first liquid and the second liquid in an unmixed state and the liquid mixture Lc.

On the other hand, in the configuration of a nozzle illustrated in Fig. 19 of JP 2009-131590 A, that is, in a mixing unit body 100 illustrated in Fig. 3 (described below), although the gas G is fed to a confluent section from the periphery, the gas G is not fed thereto from the rear end side. Thus, liquid in the confluent section is not pushed out toward the tip side, and may remain in the confluent section.

In the applicator 10, the liquid mixture Lc can be reliably jetted from the tip 24a of the nozzle head 24 by the operation of the pressing unit 36. Further, even when the liquid mixture Lc is jetted once and then again jetted after a predetermined time, clogging does not occur. Thus, even when the liquid mixture Lc is repeatedly and intermittently jetted, a reduction in the flow rate of the gas G is prevented. Further, it is possible to prevent an increase in the sliding resistance of the pressing unit 36, that is, an increase in force required for pushing out the liquid mixture Lc from the liquid feed unit. Furthermore, since a reduction in the gas flow rate of the gas G is prevented, mixing failure between the first liquid and the second liquid can also be prevented. Thus, even when the liquid mixture Lc is intermittently jetted, a liquid mixture with an appropriate mixture ratio can be jetted at a constant jetting amount.

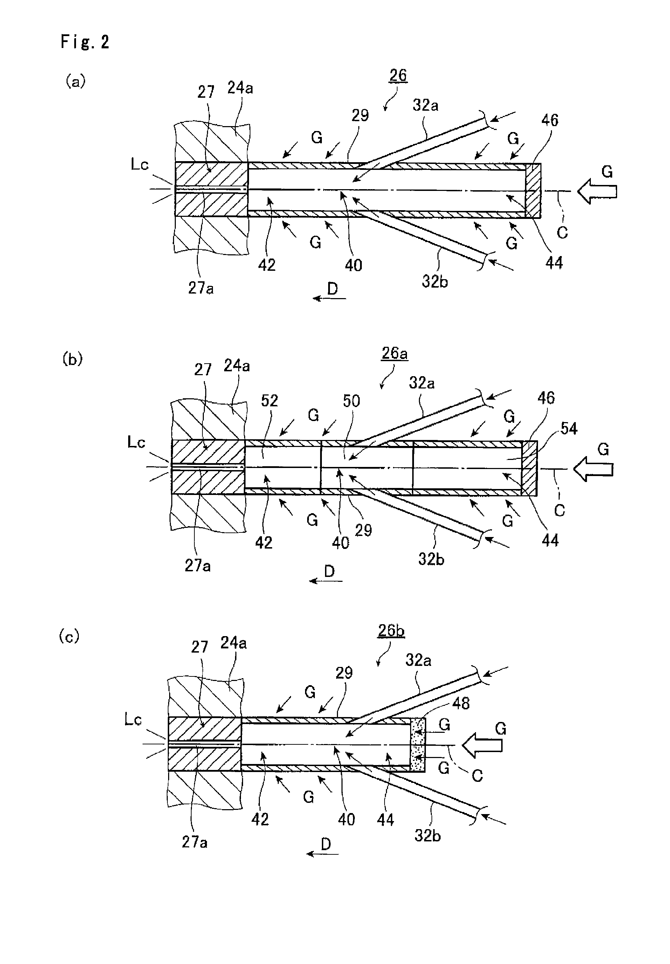

In the present embodiment, the configuration of the mixing unit body 26 of the nozzle head 24 is not limited to the configuration illustrated in Fig. 2(a). For example, as in a mixing unit body 26a illustrated in Fig. 2(b), a confluent section 40, a stirring section 42, and an introduction section 44 may include separate members. The mixing unit body 26a illustrated in Fig. 2(b) has a configuration similar to the configuration of the mixing unit body 26 of Fig. 2(a) except that the confluent section 40 is impermeable to the gas G. Thus, detailed description thereof will be omitted.

In the mixing unit body 26a illustrated in Fig. 2(b), for example, the confluent section 40 includes a gas impermeable tubular confluent member 50, the stirring section 42 includes a gas permeable tubular stirring member 52, and the introduction section 44 includes a gas permeable tubular introduction member 54. A sealing section 46 is formed on the rear end of the introduction member 54 which constitutes the introduction section 44. The gas impermeable confluent member 50 is made of a material having a lower gas permeability than the gas permeable members, for example, resin such as vinyl chloride. The gas permeable stirring member 52 and the gas permeable introduction member 54 include, for example, gas permeable tubes which are the same as the mixing unit body 26.

In the mixing unit body 26a, the first liquid and the second liquid fed to the confluent section 40 are mixed together, and mixed and stirred for gelling by gas G flowing into the stirring section 42 in the same manner as in the mixing unit body 26. Further, the liquid mixture Lc and the like in the confluent section 40 are allowed to flow toward the tip side by gas G flowing from the introduction section 44. Accordingly, the liquid mixture Lc is jetted from the tip 24a of the nozzle head 24 in the same manner as in the mixing unit body 26 illustrated in Fig. 2(a). In addition, the liquid mixture Lc is prevented from remaining inside the mixing unit body 26. Thus, even when the liquid mixture Lc is repeatedly and intermittently jetted as described above, a reduction in the flow rate of the gas G is prevented. Further, it is possible to prevent an increase in the sliding resistance of the pressing unit 36, that is, an increase in resistance required for feeding the liquid mixture Lc to a target region.

Further, the mixing unit body may have the configuration of a mixing unit body 26b illustrated in Fig. 2(c). The mixing unit body 26b illustrated in Fig. 2(c) has a configuration similar to the configuration of the mixing unit body 26 of Fig. 2(a) except that an introduction section 44 is shorter than the introduction section 44 of the mixing unit body 26 illustrated in Fig. 2(a) and a permeable film section 48 which is permeable to the gas G is provided instead of the sealing section 46 disposed on the rear end of the introduction section 44. Thus, detailed description thereof will be omitted.

The configuration of the permeable film section 48 is not particularly limited to any configuration as long as it is permeable to the gas G. For example, at least one hole for allowing the gas G to pass therethrough may be provided. The size, the position, and the number of holes for allowing the gas G to pass therethrough are appropriately determined in advance by, for example, an experiment so as to prevent the liquid mixture Lc and the like from remaining inside the mixing unit body 26b.

In the mixing unit body 26b illustrated in Fig. 2(c), the first liquid and the second liquid fed to a confluent section 40 are mixed with gas G flowing into the confluent section 40 and a stirred section 42 and stirred. Further, the liquid mixture Lc in the confluent section 40 is allowed to flow toward the tip side and discharged by gas G flowing from the end of the introduction section 44.

Accordingly, the liquid mixture Lc is jetted from the tip 24a of the nozzle head 24 in the same manner as in the mixing unit body 26 illustrated in Fig. 2(a). In addition, the liquid mixture Lc is prevented from remaining inside the mixing unit body 26. Thus, even when the liquid mixture Lc is repeatedly and intermittently jetted as described above, a reduction in the flow rate of the gas G is prevented. Further, it is possible to prevent an increase in the sliding resistance of the pressing unit 36, that is, an increase in resistance required for feeding the liquid mixture Lc to a target region.

In the mixing unit body 26b illustrated in Fig. 2(c), for example, the confluent section 40 may be made of a material having a lower gas permeability than the gas permeable members, for example, vinyl chloride as with the mixing unit body 26a illustrated in Fig. 2(b). Also in this case, an effect similar to the effect of the mixing unit body 26 illustrated in Fig. 2(a) can be obtained.

In the confluent section 40, the first liquid and the second liquid may be mixed together in addition to the confluence.

Further, in any of the mixing unit bodies, the tip member 27 may be directly connected to the confluent section 40 without providing the stirring section 42. In this case, the liquids are allowed to join together and further mixed in the confluent section 40 and the liquid mixture Lc is jetted through the opening 27a of the tip member 27.

The applicator 10 of the present embodiment can be used in laparoscopic surgery and abdominal surgery. The applicator 10 is capable of applying, for example, an antiadhesive material and a biological tissue adhesive to a target region such as organs and the abdominal wall.

The applicator 10 is inserted into the abdominal cavity through a trocar previously placed on the abdominal wall.

As the trocar, various known trocars used in laparoscopic surgery can be used. For example, a trocar tube disclosed in Fig. 1 of JP 2009-226189 A can be used.

Further, in the applicator 10, the nozzle head 24 may have a curved configuration. In this case, for example, the sheath 14 restricts the shape of the curved part of the nozzle head 24, and an antiadhesive material or a biological tissue adhesive can be applied to a target region with the nozzle head 24 curved.

The present invention is basically configured in the above manner. In the above, the applicator of the present invention has been described in detail. However, it is needless to say that the present invention is not limited to the above embodiment and various improvements and modifications may be made without departing from the gist of the invention.

### Example

Hereinbelow, the effect of the applicator of the present invention will be more specifically described. The applicator of the present invention is not limited to an example described below.

In the present example, in order to confirm the effect of the applicator of the present invention, applicators of Example 1 and Comparative Example 1 provided with nozzle heads having different structures (described below in detail) were manufactured, and the gas flow rate and resistance required for spraying were measured in each of the applicators of Example 1 and Comparative Example 1. The result of the measurement is shown in Table 1.

First, Example 1 and Comparative Example 1 will be described.

Example 1 has a configuration similar to the applicator 10 illustrated in Fig. 1 having the mixing unit body 26 illustrated in Fig. 2(a). Thus, detailed description of Example 1 will be omitted. A cylindrical gas permeable PTFE tube having a total length of 35 mm is used as the mixing unit body 26. The attachment positions of the first inner tube 32a and the second inner tube 32b are 15 mm from the tip in the axial direction D of the mixing unit body 26.

In the applicator of Example 1, the length of the nozzle body 22 is 30 cm, the outer diameter of the nozzle body 22 is 3.7 mm, and the inner diameter of the sheath 14 is 4.5 mm. Further, air is used as the gas.

The applicator of Comparative Example 1 has a nozzle having a configuration similar to the configuration illustrated in Fig. 19 of Patent Literature 1, specifically, has a mixing unit body 100 of a nozzle head having a configuration illustrated in Fig. 3. Except for this point, the configuration of the applicator of Comparative Example 1 is similar to the configuration of the applicator of Example 1. Thus, for Comparative Example 1, the configuration of the mixing unit body 100 will be described in detail and detailed description of the other configurations will be omitted.

A cylindrical gas permeable PTFE tube having a total length of 35 mm is used as the mixing unit body 100 of Comparative Example 1 illustrated in Fig. 3. A tip member 27 is connected to the tip of the mixing unit body 100. A first inner tube 32a and a second inner tube 32b are connected to an opening on the rear end of the mixing unit body 100.

In Comparative Example 1, a first liquid and a second liquid are fed from the rear end of the mixing unit body 100. The first liquid and the second liquid are mixed together and stirred by gas G flowing into a confluent section of the first liquid and the second liquid from a peripheral face 100a of the mixing unit body 100, and jetted as a liquid mixture Lc through an opening 27a of the tip member 27.

Next, a measurement device used in measurement of the gas flow rate and the resistance required for spraying and a method of measuring the gas flow rate and the resistance in the present example will be described.

Fig. 4 is a schematic view illustrating the measurement device used in the method of measuring the gas flow rate and the resistance in the applicator. In Fig. 4, part of the applicator 10 is not illustrated.

The measurement device 110 illustrated in Fig. 4 includes an autograph 112, a gas flow meter 114, a regulator 116, and a gas feed unit 118.

The applicator 10 is attached to the autograph 112 by fixing the syringes 34a and 34b by a fixing jig 120.

The autograph 112 pushes the pusher 35a of the syringe 34a and the pusher 35b of the syringe 34b through a rod-like jig 122 (hereinbelow, referred to as the pushing jig 122) and records a force generated during the pushing as the resistance. When a gel liquid mixture is present inside the nozzle head, that is, when the nozzle head is clogged, the gel liquid mixture becomes resistance when the pushers 35a and 35b are pushed. Thus, the resistance during the pushing is an index which indicates the degree of clogging.

The gas feed unit 118 is connected to the port 23 of the applicator 10 through the gas flow meter 114 and the regulator 116. The gas flow rate in the gas flow meter 114 is defined as the gas flow rate of the applicator. When a gel liquid mixture is present inside the nozzle head, that is, the nozzle head is clogged, the gas flow rate also decreases from an initially set gas flow rate. Thus, a change in the gas flow rate from the set gas flow rate is an index which indicates the degree of clogging.

A precision universal tester (AG-X 1kN (model) manufactured by SHIMADZU CORPORATION) was used as the autograph 112. A flow meter (8500MM-0-1-1 (model) manufactured by KOJIMA INSTRUMENTS INC.) was used as the flow meter 114. The gas feed unit 118 is a gas cylinder filled with air.

Hereinbelow, the method of measuring the gas flow rate and the sliding resistance in the applicator will be described.

First, 3.6 ml of distilled water was dropped into powder A using a micropipette, and thereafter sufficiently stirred to dissolve the powder A. Further, 1.55 ml of distilled water was dropped into powder B using a micropipette, and thereafter sufficiently stirred to dissolve the powder B.

As the powder A, powder of 1.25 g of NHS dextrin and 1.25 g of trehalose was used. As the power B, power of 0.042 g of sodium bicarbonate and 0.11 g of sodium carbonate was used.

After the dissolving, a solution of the powder A and a solution of the powder B were respectively filled into the syringe 34a and the syringe 34b. After the filling, a priming operation was performed to remove air inside the syringes 34a and 34b.

Then, a check valve was attached to the tip of each of the syringes 34a and 34b and the solution was filled up to the tip thereof. Then, the syringes 34a and 34b were connected to the applicator of Example 1 or the applicator of Comparative Example 1 and attached to the autograph 112 through the fixing jig 120.

Then, the gas flow meter 114 was connected to the port 23, and the regulator 116 and the gas feed unit 118 were further connected thereto.

The pushing jig 122 was attached to a load cell of the autograph 112, and lowered until the pushing jig 122 makes contact with the pushers 35a and 35b.

Then, the pressure of gas from the gas feed unit 118 was regulated by the regulator 116 to set the gas flow rate at 1.5 L/min.

Then, the pushers 35a and 35b were pushed by 7.6 mm at a compression rate of 40 mm/min by the autograph 112 to perform a priming operation.

After the priming operation, the gas flow rate was measured. Then, the pushers 35a and 35b were pushed by 3.8 mm to jet the liquid mixture Lc, and the resistance during the pushing was measured. Accordingly, the first measurement was finished.

After the pushers 35a and 35b were pushed by 3.8 mm, the pushing jig 122 was released from the pushers 35a and 35b, and this state was held for one minute. Then, the pushing jig 122 was again brought into contact with the pushers 35a and 35b, and the gas flow rate was measured. Then, the pushers 35a and 35b were again pushed by 3.8 mm, and the resistance during the pushing was measured. Accordingly, the second measurement was finished.

After the second measurement, the pushing jig 122 was released from the pushers 35a and 35b, and this state was held for one minute. Then, the gas flow rate and the resistance during the pushing were again measured in the same manner as in the first measurement, and the third measurement was finished.

Then, the pushing jig 122 was released from the pushers 35a and 35b, and this state was held for one minute. Then, the gas flow rate and the resistance during the pushing were measured in the same manner as in the first measurement, and the fourth measurement was finished. Then, the pushing jig 122 was released from the pushers 35a and 35b, and this state was held for one minute. Then, the gas flow rate and the resistance during the pushing were measured in the same manner as in the first measurement, and the fifth measurement was finished. In this manner, the measurement of the gas flow rate and the resistance during the pushing was repeatedly performed with the one-minute holding with the pushing jig 122 released from the pushers 35a and 35b therebetween, and intermittent spraying was performed five times in total after the priming. The gas flow rate was measured before the measurement of the resistance in each time, and the resistance during the pushing was measured.

**[Table 1]**

| | Number of spray times | Gas flow rate (l/min) | Resistance (n) |
|---|---|---|---|
| Example 1 | 1 | 1.51 | 27.15 |
| | 2 | 1.51 | 26.93 |
| | 3 | 1.49 | 27.46 |
| | 4 | 1.52 | 27.12 |
| | 5 | 1.49 | 27.03 |
| Comparative Example 1 | 1 | 1.25 | 30.71 |
| | 2 | 1.19 | 32.50 |
| | 3 | 1.13 | 36.52 |
| | 4 | 1.06 | 39.36 |
| | 5 | 0.84 | 40.57 |

As shown in Table 1, in Example 1, even when the spraying is repeatedly performed five times, the gas flow rate hardly changes from the set gas flow rate (1.5 L/min). In Example 1, there is also little change in the resistance even when the spraying is repeatedly performed five times.

On the other hand, in Comparative example 1, when the spraying is repeatedly performed five times, the gas flow rate decreases from the set gas flow rate (1.5 L/min) in each time. Further, when the spraying is repeatedly performed five times, the resistance increases in each time.

The result shows that, in the configuration of the present invention, clogging is not caused by the liquid mixture even when the liquid mixture is intermittently sprayed.

### Reference Signs List

- 10: applicator
- 12: nozzle
- 14: sheath
- 20: spray head
- 22: nozzle body
- 24: nozzle head
- 26, 26a, 26b, 100: mixing unit body
- 30b: second connection section
- 30a: first connection section
- 34a: first syringe
- 34b: second syringe
- 36: pressing unit
- 38: gas feed unit
- 40: confluent section
- 42: stirring section
- 44: introduction section
- 46: sealing section
- 48: permeable film section
- 50: confluent member
- 52: stirring member
- 54: introduction member
- 110: measurement device
- Lc: liquid mixture

## Claims

1. An applicator comprising:
a nozzle including a nozzle head located at a tip thereof and an inner part to which gas is fed;
at least two inner tubes inserted into the nozzle and used for feeding at least two kinds of liquids that gel when mixed together to the nozzle head; and
a liquid feed unit feeding each of the liquids to each of the inner tubes, wherein
the nozzle head includes a mixing unit body to which the inner tubes are connected and inside which the liquids are allowed to join and mixed together and an opening formed on a tip side of the mixing unit body and used for jetting a liquid mixture of the liquids together with the gas, and
the gas is fed from at least a rear end side with respect to a confluent section in which the liquids join together in the mixing unit body.

2. The applicator according to claim 1, wherein the mixing unit body is provided with a gas introduction section which allows the gas to pass therethrough and feeds the gas to the confluent section from at least the rear end side, the gas introduction section being located on the rear end side of the confluent section.

3. The applicator according to claim 1 or 2, wherein the mixing unit body is further provided with a stirring section which allows the gas to pass therethrough and at least stirs the liquid mixture, the stirring section being located between the confluent section and the opening.

4. The applicator according to any one of claims 1 to 3, wherein the gas introduction section includes a tubular gas permeable member and an end on the rear end side of the gas introduction section is sealed.

5. The applicator according to any one of claims 1 to 4, wherein the gas introduction section includes a member having at least one hole formed for allowing the gas to pass therethrough.

6. The applicator according to any one of claims 1 to 5, wherein the confluent section includes a tubular member, and the inner tubes are connected to a peripheral face of the confluent section at positions symmetric with respect to an axis in a longitudinal direction.

7. The applicator according to any one of claims 1 to 6, wherein the confluent section is formed of a material having a lower gas permeability than the gas permeable member.
